(19) Europäisches Patentamt
European Patent Office
Office européen des brevets

(11) Publication number: **0 086 538**
**B1**

# (12) EUROPEAN PATENT SPECIFICATION

(45) Date of publication of patent specification: **07.08.85**

(51) Int. Cl.[4]: **C 07 C 9/04,** C 07 C 1/04, C 07 C 1/10, B 01 J 23/74

(21) Application number: **83200208.3**

(22) Date of filing: **09.02.83**

## (54) Process for the production of methane.

(30) Priority: **12.02.82 NL 8200544**

(43) Date of publication of application:
**24.08.83 Bulletin 83/34**

(45) Publication of the grant of the patent:
**07.08.85 Bulletin 85/32**

(84) Designated Contracting States:
**BE DE FR GB IT NL**

(56) References cited:
**EP-A-0 028 835**
**DE-A-1 767 202**
**DE-C- 376 428**
**FR-A-1 074 037**
**FR-A-2 256 234**
**FR-A-2 347 326**
**GB-A-1 367 088**

**RECUEIL DES TRAVAUX CHIMIQUES DES PAYS-BAS, vol. 70, September-October 1951, pages 793-812, The Hague, NL. J.J.B. VAN EIJK VAN VOORTHUIJSEN et al.: "Structure and properties of compounds formed during the preparation of nickel-on-silica catalysts"**

(73) Proprietor: **VEG-GASINSTITUUT N.V.**
**Wilmersdorf 50 P.O. Box 137**
**NL-7300 AC Apeldoorn (NL)**

(72) Inventor: **Kuijpers, Eugène Gérard Marie, Dr.**
**Topaasstraat 15**
**NL-7314 HS Apeldoorn (NL)**
Inventor: **Geus, John Wilhelm**
**Gezichtslaan 100**
**NL-3723 GJ Bilthoven (NL)**
Inventor: **Bottelberghs, Pierre Henri, Dr.**
**Utrechtseweg 93**
**NL-3818 EC Amersfoort (NL)**

(74) Representative: **van der Beek, George Frans, Ir. et al**
**Nederlandsch Octrooibureau P.O. Box 29720**
**NL-2502 LS The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of methane from gas mixtures containing hydrogen and/or steam and at least 5% by volume of carbon monoxide by means of a nickel catalyst, which contains metallic nickel particles as an active catalyst component on a thermally stable oxidic carrier.

On account of the high oil prices a growing interest is being displayed in the production of methane from gas mixtures containing carbon monoxide, which can be obtained, for example, from coal. It is possible in this way to produce a substitute for natural gas from coal. The natural gas currently distributed in many countries contains about 85% by vol. of methane. Hence, if it is desired to produce gas from coal, which is readily interchangeable with the currently distributed natural gas, such gas must have a high methane content.

The obvious method for production of methane from coal would seem to be direct hydrogenation of coal according to the reaction:

$$C+2H_2\rightleftharpoons CH_4.$$

However, with many types of coal such a direct hydrogenation process presents technical problems, while furthermore the reaction rate is too low to render such hydrogenation technically feasible. When the temperature is raised, the above equilibrium shifts to the left. If it is desired to increase the reaction rate by raising the temperature, it is necessary to apply high hydrogen pressures. Even at a high temperature and elevated hydrogen pressure the reaction rate is still too low. Moreover, hydrogen is required for direct hydrogenation. It is true that the production of hydrogen from coal has been satisfactorily achieved technically, but such production requires much thermal energy. The difficult reaction between coal and hydrogen as well as the high energy costs, do not make direct hydrogenation of coal an attractive proposition yet.

Another known process is the gasification of coal at elevated temperature with steam and oxygen. At temperatures between 1000 and 1500°C coal mainly reacts according to the reaction equation:

$$C+H_2O\rightleftharpoons CO+H_2,$$

which reaction is highly endothermic.

The required heat of reaction is derived from the reaction which takes place according to the equation

$$2C+O_2\rightleftharpoons 2CO,$$

which reaction is highly exothermic.

If in the gasification of coal air is used as the source of oxygen, the gas obtained is diluted by the nitrogen which remains after the reaction of the oxygen.

However, dilution with nitrogen is generally not admissible. For this reason it is necessary to work with pure oxygen in the production of so-called synthetic natural gas. Inasmuch as the production or pure oxygen requires a lot of equipment and energy, oxygen consumption plays an important part in the economics of the gasification process.

Another important factor in coal gasification is other energy utility consumption. Gasification processes can be distinguished according to their operating temperature. If gasification takes place at a relatively low maximum temperature, the oxygen consumption is indeed low, but a lot of steam is required, e.g. from 5 to 10 times the stoichiometrically required quantity of steam. This higher steam consumption is compensated by the lower oxygen consumption. At lower temperature the following equilibria shift somewhat to the right:

$$C+2H_2\rightleftharpoons CH_4$$

$$CO+H_2O\rightleftharpoons CO_2+H_2$$

Partly on account of the limited quantity of oxygen supplied the $H_2/CO$ ratio of the gas obtained at relatively low temperature is about 2.

If more oxygen is supplied during gasification of coal, the operating temperature will be higher and less steam is required to allow the coal to react, e.q. only 1.3 times the stoichiometrically required quantity of steam. In the gas thus obtained the $H_2/CO$ ratio is much lower, viz. between 0.4 and 0.8.

It is an object of the present invention to produce methane from gas mixtures containing carbon monoxide.

The mixtures obtained in the gasification of coal can be used as such gas mixtures. The conversion of carbon monoxide into methane can take place according to the reaction:

$$CO+3H_2\rightleftharpoons CH_4+H_2O.$$

It is known that this reaction proceeds at a high rate when nickel catalysts are used. However, when using gas mixtures obtained in the gasification of coal as a starting material, the problem which arises is that for the methanation reaction the $H_2/CO$ ratio must be 3, while in coal gasification, as mentioned above, gas mixtures are obtained with a $H_2/CO$ ratio of from 0.4 to 2. Thus, even the $H_2/CO$ ratio obtained with a low gasification temperature and a lot of steam is too low for methanation. Moreover the methanation reaction is highly exothermic. As it is difficult to dissipate the heat of reaction quickly, the temperature rises considerably. This can cause damage to the reactor, while often the catalyst is deactivated by the high temperature. Furthermore, at high temperature the equilibrium shifts to the left, as a result of which the conversion of carbon monoxide is seriously restricted.

It is possible to itself to adjust the $H_2/CO$ ratio of the gas mixture according to the so-called carbon-monoxide shift reaction:

$$CO+H_2O \rightleftharpoons CO_2+H_2.$$

This reaction requires a catalyst. In a lot of cases a copper/zinc-oxide catalyst is used for this purpose, but this catalyst is highly sensitive to sulphur compounds. Iron-oxide/chromium-oxide catalysts can also be used. They are less sensitive to sulphur compounds, but on the other hand their activity is lower. For this reason it is generally desirable to remove the sulphur compounds from the gas mixture prior to conversion of the carbon monoxide with steam. However, this renders the gas treatment cumbersome, because the sulphur compounds, hydrogen sulphide in particular, are removed by scrubbing with liquids. To this end the gas must be cooled to about 90°C, which causes the steam which is present in the gas to condense. The carbon-monoxide shift reaction, however, requires an excess of steam to shift the equilibrium to the right. When removing the sulphur compounds, however, the steam content of the gas drops considerably. If it is desired to cause the shift reaction to take place after removal of the sulphur compounds, steam must once more be supplied, while moreover the reaction mixture must be raised from 90°C to the inlet temperature of the shift reactor, i.e. to between 350 and 450°C. All these measures decrease the thermal efficiency of the gas production.

In the event of a catalyst being used for the carbon-monoxide shift reaction, which is resistant to sulphur compounds, e.g. on a basis of cobalt oxide and molybdenum oxide, the shift reaction is applied prior to removal of the sulphur compounds. But in this case as well serious difficulties are still encountered. In the majority of methods involving the removal of sulphur compounds (hydrogen sulphide in particular) carbon dioxide is also formed. The result is that the sulphur compounds become available with a large excess of carbon dioxide. It is technically difficult, however, to convert highly diluted hydrogen sulphide into elementary sulphur according to the Claus process, which is almost invariably used for this purpose. In the presence of a large volume of inert gas the initial reaction of the present-day Claus process proceeds ineffectively:

$$2H_2S+3O_2 \rightleftharpoons 2SO_2+2H_2O.$$

The reason for this is that, when substantially diluted with inert gas, the gas mixture does not reach the temperature required for the reaction. Since the carbon dioxide content increases due to the carbon monoxide shift reaction, the concentration of hydrogen sulphide in the gas formed drops considerably after the previous conversion of the carbon monoxide.

In methods involving gasification of coal at high temperature with little steam and much oxygen there is no objection to the sulphur compounds being removed immediately after gasification. The thermal efficiency is not greatly influenced by the condensation of the slight excess of steam. Gasification at higher temperature, however, produces a relatively large volume of carbon monoxide. After removal of the sulphur compounds a lot of steam is then still required for conversion into hydrogen, viz. about 2 mol steam per mol carbon monoxide to be converted.

It is evident, therefore, that the combination of sulphur removal and the carbon monoxide shift reaction leads to difficulties. When coal is gasified with a large volume of steam at a low temperature, it is necessary to use a carbon monoxide shift catalyst which is resistant to sulphur, while the processing of the hydrogen sulphide formed is difficult on account of the large volume of carbon dioxide present.

In case of gasification of coal at a higher temperature with little steam more carbon monoxide must be converted, which takes steam, while more heat of reaction must be dissipated at low temperature in order to shift the equilibrium of the shift reaction in the desired direction.

The difficulties encountered in the dissipation of the substantial heat of the methanation reaction have hitherto not been satisfactorily solved either. In the methanation reaction the temperature of the reaction mixture rises quickly as a result of the substantial heat of reaction, where the reaction in the catalyst bed starts to proceed considerably. As a result of the fast rising temperature the reaction rate also increases substantially, until the reaction does not proceed any further owing to the position of equilibrium. The result is that methane is formed in only a very limited part of the catalyst bed. In the part of the catalyst bed where methane is formed, however, the catalyst also loses its activity fairly quickly. As a result the zone in which the temperature rises quickly, shifts along the reaction and the catalyst bed must be regenerated or renewed after a short time.

A solution to this problem was proposed by G. A. White, T. R. Roszkowski and D. W. Stanbridge in Advances in Chemistry, Series *146* (1975), pp. 138—148. Six reactors with catalyst beds in series are employed in this proposed process. The gas mixture is cooled after each reactor before it is passed to the following reactor. However, the outlet gas mixture is not just passed to the first reactor, but also partly added to the gas leaving the first reactor and to the gas leaving the second reactor. Finally, steam is added as a diluent in order to get beyond the thermodynamical carbon deposition boundary. The use of many reactors with their coupled heat-exchangers requires substantial investments, while dilution with steam costs thermal energy.

In order to achieve dissipation of the heat of reaction it is also proposed to recirculate part of the reaction mixture over the catalyst bed. The mixture is cooled after each pass through the reactor. An objection to this procedure is that even with very high feed rates through the reactor the reaction is difficult to control. A slight variation in the gas rate can cause the reactor temperature to rise quickly in places. This in turn results in the catalyst being deactivated, while damage to the reactor can also occur. For this reason it was proposed to combine recirculation with the use of a number of reactors, using the methane formed as diluent, while the feed is divided over a number of reactors connected in series.

These solutions, however, do not produce satisfactory results and are expensive as regards both investment and energy consumption. Nor did it prove very well possible to dissipate the heat by placing the catalyst in effective thermal contact with cooling ribs in the reactor. The commonly used heterogeneous catalysts are not suitable for this purpose. These catalysts have a high porosity and thus possess a low heat-conducting capacity.

A fluidized bed reactor is the most suitable to remove the heat of reaction from the reactor. A. Anderlohr and K. Hedden describe a process in GWF-Gas/Erdgas *118* (1977), pp. 422—426, for methanation and simultaneous conversion of gases rich in carbon monoxide by passing these gases over a nickel catalyst in a fluidized bed. With this method the removal of the thermal energy from the reactor proceeds at a fast rate. Nothing is said here about the nature of the catalyst. In the method described above the catalyst used must not only be active and thermally stable, but it must also be resistant to wear. In the case of a fluidized bed reactor it is furthermore difficult to prevent spreading of the residence time and thus incomplete conversion.

Accelerated carbon deposition is described by W. Lommerzheim and K. Hedden (1st Gas Research Conference, Chicago, page 49) for the case that the $H_2$/CO ratio is decreased from 4 down to 1. These authors mention the rapid abrasion of their catalyst in a fluidized bed when the $H_2$/CO ratio is decreased as mentioned. An enhanced formation of fines was observed at low $H_2$/CO ratios. Apparently carbon deposition decreases the mechanical stability of the catalyst. To avoid carbon deposition steam must be added when the $H_2$/CO ratio lies below 1.5.

It is still not known how deactivation of the catalysts used for methanation can be effectively counteracted. Even if a substantial excess of hydrogen is applied, deactivation of the catalyst takes place as a result of carbon deposits. According to H. H. Gierlich, M. Fremary, A. Skov and J. R. Rostrup-Nielsen in "Catalyst Deactivation", Elsevier, Amsterdam (1980), p. 459, a fast temperature rise is observed locally in the reactor when a gas mixture consisting of 10,4% by vol. $CO_2$, 8,6% by vol. CO, 72,5% by vol. $H_2$ and 0,5 by vol. $N_2$ is passed over a nickel catalyst. This temperature profile shifts through the reactor as the reaction proceeds. Despite the excess of hydrogen it appeared that carbon had been deposited on the catalyst. This deactivation through carbon deposits proved to take place at a temperature as low as between 300 and 350°C, although the final temperature in the reaction was between 600 and 700°C. This implies that restriction of the temperature rise by fast recirculation or substantial dilution still cannot completely avoid carbon being deposited.

P. K. Agrawal, W. D. Fitzharris and J. R. Katzer describe in "Catalyst Deactivation", Elsevier, Amsterdam (1980), p. 179, that when passing gas mixtures containing 1 and 4% by vol. CO in hydrogen respectively, over a nickel film deposited by evaporation, whilst using a very high recirculation ratio, slow deactivation of the catalyst occurs nevertheless at a temperature of 275°C.

It is known from DE—A—26 06 755 that catalysts comprising exlusively nickel as active component, applied on a carrier, are quickly deactivated during the methanation reaction at $H_2$/CO ratios of between 1:1 and 10:1 and at temperatures of between 400 and 600°C.

For this reason it has been suggested to use catalysts containing copper and molybdenum in addition to nickel as active components. Although catalysts of this kind are deactivated less quickly, their initial activity is lower than that of catalysts containing nickel only as an active component.

The deactivation of nickel catalysts in the methanation reaction is also described in DE—A—25 51 958. Carbon deposits are observed at temperatures above 400°C. To counteract this deposition of carbon it is suggested to add hydrogen sulphide to the reaction mixture. Although adsorption of sulphur compounds poisons the nickel surface, this would allegedly counteract carbon deposits to such an extent that the ultimate stable activity is higher than without the addition of hydrogen sulphide. Nevertheless, adsorption of hydrogen sulphide decreases the activity of the catalyst considerably.

It is also known from DE—A—26 24 396 that in the methanation reaction carbon is deposited on nickel catalysts. Excess of hydrogen is used; for example, a gas mixture is described containing 7.46% by vol. CO and 35.57% by vol. $H_2$, while according to another example 1% by vol. CO in hydrogen is used.

When feeding the gas to the catalyst the temperature is between 230 and 270°C, while after passing

through the catalyst bed the temperature is between 580 and 610°C. In order to counteract carbon deposits the use of nickel-molybdenum catalysts is suggested, which are not subject to rapid deactivation.

It is evident from the above review of the literature that in the production of methane from gas mixtures containing carbon monoxide, which have been obtained, for example, by gasification of coal with oxygen and steam, the removal of catalyst-poisoning compounds, such as sulphur compounds, the carbon monoxide shift reaction and the substantial heat of reaction in methanation present difficulties, which have hitherto not been solved satisfactorily.

In order to solve these problems it is necessary to consider the three processes integrally. There is little sense in improving only one of the elements, e.g. the methanation reaction.

The difficulties mentioned in connection with the removal of catalyst-poisoning compounds in the carbon monoxide shift reaction and in methanation might be solved, if an effective and stable catalyst could be found, suitable for the reaction:

$$2CO+2H_2 \rightleftharpoons CO_2+CH_4.$$

Whereas hitherto, as explained above, the customary methanation process required a $H_2/CO$ ratio of 3, the above mentioned reaction requires a $H_2/CO$ ratio of only 1.

Gasification of coal with a lot of steam and little oxygen produces a raw gas with a $H_2/CO$ ratio of about 2. Sulphur compounds can be separated from the gas and the excess of steam condensed, whereupon the gas can be passed over the catalyst, which accelerates the above reaction.

As no shift reaction is used, the gas passed into the sulphur-removal section contains less carbon dioxide. This renders the conversion of the hydrogen sulphide formed to elemental sulphur easier. If the desired catalyst were available, the difficulties encountered in the processing of raw gas with a $H_2/CO$ ratio of more than 1 would be essentially solved.

In the gasification of coal at a higher temperature with more oxygen and less steam the above reaction is not possible without appropriate measures. The excess of carbon monoxide reacts according to Boudouard's reaction:

$$2CO \rightleftharpoons C+CO_2.$$

The carbon formed can deactivate the catalyst, while carbon deposits in the reactor can give rise to plugging. When processing a raw gas with a $H_2/CO$ ratio of less than 1, it is necessary to use a catalyst, which also accelerates the following reaction:

$$4CO+2H_2O \rightarrow 3CO_2+CH_4.$$

After removal of the sulphur compounds it is then only necessary to add a slight quantity of steam to the purified gas. In addition to the reaction:

$$2CO+2H_2 \rightarrow CO_2+CH_4$$

the following reaction proceeds over the catalyst:

$$4CO+2H_2O \rightarrow 3CO_2+CH_4.$$

Thus, the shift reaction can also be omitted in cases where the gas mixture used for methane production has a low $H_2/CO$ ratio. This improves the thermal efficiency of the methane production from coal, while lower investments in equipment are necessary.

In the production of methane according to the method indicated above the difficulties with respect to the substantial heat of reaction evolved during methanation are also reduced.

The following three reaction equations can be drawn up for methane formation:

| | |
|---|---|
| $4CO+12H_2 \rightarrow 4CH_4+4H_2O$ | H=−824 kJ |
| $4CO+4H_2 \rightarrow 2CO_2+2CH_4$ | H=−494 kJ |
| $4CO+2H_2O \rightarrow 3CO_2+CH_4$ | H=−329 kJ |

Per molecule of carbon monoxide converted the heat evolved during the second and third reactions is much less than in the first reaction, i.e. the "conventional" methanation reaction. This means that the thermal energy liberated in the reactor per unit of volume is greatly reduced at the point where the reaction sets on. As a result the temperature of the reaction mixture rises less quickly, thus causing the reaction rate to rise more gradually. Consequently, the heat of reaction is liberated in a larger reactor volume, the local temperature rise is kept down and the catalyst deactivation is reduced.

In the foregoing attention was drawn on a number of occasions as to the risk of carbon being deposited on the catalyst. It is known that in a lot of cases mixtures of hydrogen and carbon monoxide are processed under conditions which make the carbon deposition thermodynamically probable. In J. Chem. Soc. (1929),

pp. 1903—1913, it was described even then that the rate at which graphite is formed is very low. In the case of carbon being formed this substance occurs in a thermodynamically far less stable form. Hence, it is possible to operate in a range where graphite is thermodynamically stable, provided any carbon formed is quickly converted into gaseous products before the slow transition into the more stable graphite can take place.

In the previously mentioned article in GWF-Gas/Erdgas *118* (1977), pp. 422—426, it is stated that in the conversion of synthetic gases at a temperature of 430°C increasing carbon deposits are observed when the $H_2/CO$ ratio is reduced from 3 to 1.5. When lowering this ratio to 1, the filter present in the reactor is blocked with soot.

Several publications are also known, in which it is stated that the shift reaction and the methanation reaction can occur simultaneously on a catalyst.

Reference may be made to a publication by B. J. Koch, H. Yoon and W. B. Carter in Coal Tech., Houston 1979, pp. 52—80, in which the above mentioned reactions are discussed. However, no information is given about the catalyst or its stability nor of the composition of the gas mixture being processed.

The combination of the shift and methanation reactions is discussed in more detail in an article in Hydrocarbon Processing of April 1981, pp. 114—118 and the US—A—4,133,825. It is known from these references that a synthesis gas containing carbon monoxide and hydrogen is simultaneously heated and saturated with water, whereupon the gas stream is divided into two or more streams. The first stream is mixed with recirculated product gas and then passed over a catalyst at 250 to 550°C. The product of this first reaction is mixed with the second stream and the mixture is again passed over a catalyst at 250 to 550°C. The product gas is partly recirculated to the first reactor. If desired, steam can also be added to the saturated primary gas. The gas mixture processed has a $H_2/CO$ ratio of 0.4. However, no information is given about the catalysts used nor about their stability.

In FR—A—2,256,234 a process has been described for the production of methane in which process a gas stream containing hydrogen and carbon monoxide in a ratio of from 0.5 to 1.15 is mixed with water to bring the molar percentage of the water in the gas stream at a volume of from 0.1 to 15.0, the resulting gas stream is preheated to a temperature in the range of from 149°C to 538°C and thereafter subjected to methanation to form a gas mixture which has a high methane content. Nothing is said in this specification about the catalyst stability towards carbon (whisker) formation. From experiments, however, it appears that the stability of the catalysts used therein is bad and will give rise to numerous problems in the operation of the process.

It is an object of this invention to improve the process for the preparation of methane from gas mixtures containing hydrogen and/or steam and at least 5% by volume of carbon monoxide in such a way, that the process can be carried out over long time periods without carbon deposition and catalyst deactivation. Another object of the invention is to improve the process in such a way that the $H_2/CO$ ratio can be selected in wide ranges and particularly with high methane production selectivity, especially if the $H_2$-content is low.

It has been found that these objects can be accomplished in a process for the preparation of methane from gas mixtures containing hydrogen and/or steam and at least 5% by volume of carbon monoxide by means of a nickel catalyst, which contains on a thermally stable oxidic carrier metallic nickel particles as an active catalyst component, if according to the invention a gas mixture, in which the molar ratio $H_2/CO$ is below 2.45, is contacted with a nickel catalyst, in which the metallic nickel particles are chemically bonded to the thermally stable oxidic carrier.

As thermally stable oxidic carriers there can be used according to the invention the compounds, which according to the state of the art are used as carriers for catalysts in many fields. Such carriers have a large specific surface area. Non-restrictive examples for such carriers are alumina, silica, magnesia, silica-alumina, silica-magnesia, zirconia, silica-zirconia, titania, silica-zirconia-titania, crystalline or amorphous alumino silicate molecular sieves and metal phosphates. According to the invention zirconia and magnesia, but especially alumina and silica are preferred.

The element or elements, the oxides of which are used as carriers according to the invention, e.g. alumina or silicon, are defined hereinafter for the sake of simplicity as carrier base element or elements.

If silica is used as a carrier, commercially available products may be used, such as Aerosil (registered trade mark). Other carriers may also be used in the form of their commercially available products.

According to a preferred embodiment of the invention the chemical bond between the metallic nickel particles and the oxidic carrier is provided by an interfacial layer different from the oxidic carrier, which layer consists of a compound containing oxygen and at least one member selected from the group consisting of nickel and the carrier base element or elements.

Generally the interfacial layer consists of

a) a compound containing nickel ions, or

b) a stable non-stoichiometric oxide of the carrier base element or elements, or

c) a compound containing nickel and the carrier base element or elements.

If the carrier is an oxide of a carrier base element or elements, which form(s) only stoichiometric oxides, the interfacial layer contains nickel ions. If, on the other hand, the carrier is an oxide of a carrier base element or elements, which can form a stable non-stoichiometric oxide, the interfacial layer comprises such non-stoichiometric oxides and may in addition comprise nickel ions.

As carrier base element or elements, which form(s) only stoichiometric oxides, preferably at least one member of the group consisting of Si, Al and Mg is used.

The interfacial layer is very thin and usually as a thickness between 0.2 and 10 nm, preferably between 0.5 and 5 nm. In fact this interfacial layer has a thickness of only a few atoms. It is extremely important that essentially all of the metallic nickel particles of the catalyst are chemically bonded to the carrier as described in the above. The metallic nickel particles of the active catalyst do not form a continuous layer, but are individual particles which are chemically bonded to the carrier.

It has been observed that, if the active catalyst contains appreciable amounts of metallic nickel particles which are not chemically bonded to the carrier, these particles will give rise to the formation of carbon whiskers in a gas atmosphere containing a high percentage of CO. Therefore, it is preferred that in the catalyst used in the process of the invention per $cm^3$ of the catalyst bed there is not more than 0.05 g, preferably not more than 0.03 g and most preferably not more than 0.01 g metallic nickel, which is not chemically bonded to the carrier, e.g. through the interfacial layer.

Preferably the nickel particles size distribution in the catalyst used in the process of the invention is such that less than 10% by volume of the particles is smaller than 2 nm and less than 10% by volume of the particles is larger than 30 nm.

Nickel is mostly precipitated on the oxidic carriers in the form of hydroxide or oxide. The hydroxide or oxide is not, however, catalytically active for the above reactions, so that it has to be reduced to nickel metal. The most obvious way of effecting a chemical bond would be partial reduction of the nickel hydroxide or nickel oxide primarily fixed on the carrier. The nickel particle thus formed is chemically bonded by the remaining nickel oxide, which in turn is bonded by the carrier. There is a problem, however, in that the reduction of a nickel oxide particle, once started, tends to proceed fast until the particle is fully reduced to nickel metal. Part of the nickel ions should be supplied in a form which is reducible more slowly than the pure oxide. To achieve this purpose the nickel ions could be reacted with the carrier into a compound, which is less readily reduced or alternatively a carrier could be used, which can be partially reduced. In the latter case reduction of the carrier material produces metal atoms, often alloyed with nickel, which strongly interact with both the nickel and the carrier.

Reaction of at least part of the nickel ions with the carrier material to form a compound which is less readily reduced than the nickel hydroxide or nickel oxide, can be achieved with $SiO_2$ as a carrier. For instance, a suspension of $SiO_2$ in a solution of nickel salts could be taken, to which an alkaline compound is added in excess over the dissolved nickel ions. If the suspension is then stirred for some time, at elevated temperature if needed, before filtering and drying the loaded carrier, the $SiO_2$ carrier reacts to some extent into nickel hydrosilicate. The portion of the $SiO_2$ reacting into hydrosilicate varies with the reactivity of the $SiO_2$ used and with the excess of alkaline compound used.

A more complete reaction of $SiO_2$ into hydrosilicate is obtained by coprecipitation of nickel and $SiO_2$, as described by Van Eijck, Van Voorthuijsen and Franzen In Rec. Trav. Chim. *70* (1951) p. 793. According to this method a solution of water glass is thoroughly mixed with a solution of nickel salt, resulting in a more or less complete reaction to form nickel hydrosilicate. Generally the nickel hydrosilicate crystallizes into relatively large, plate-shaped crystallites. Reduction of the coprecipitated material produces nickel particles which adhere very well to the remaining $SiO_2$, which still contains nickel ions. Catalysts prepared according to this method are suitable for the process of the invention.

Referring in more general terms to the methods of preparation of the nickel catalysts useful in the process of the invention it has been found that these nickel catalysts are obtainable by one of the following methods, which are given by the way of example only. These methods comprise:

a) mixing an aqueous solution of a nickel salt and an aqueous solution of a salt of the base element or elements, the oxide of which is the thermally stable oxidic carrier, raising the pH-value up to a level where the dissolved nickel and base element or elements ions have been substantially completely precipitated, ageing, if required, the precipitate in the solution, separating, hydrothermally treating, if required, and drying, calcinating and reducing the precipitate, or

b) mixing an aqueous solution of a nickel salt and an aqueous solution of a salt of the base element or elements, the oxide of which is the thermally stable oxidic carrier, with an aqueous solution of an oxalate or formate, separating, drying, calcinating and reducing the precipitate, or

c) suspending in a finely divided form the thermally stable oxidic carrier in a dilute aqueous solution of a nickel salt and precipitating a nickel compound at an elevated temperature and with vigorous agitation by forming hydroxyl ions by a chemical reaction, which is known *per se*, of compounds contained also in the solution, not faster than this is the case of hydrolysis of an aqueous solution of urea, which is present in the solution in an amount of 1 to 10 times the amount stoichiometrically required, followed by separating, calcinating and reducing the loaded carrier, or

d) suspending in a finally divided form the thermally stable oxidic carrier in a dilute aqueous solution of a nickel salt, raising the pH value of the resulting suspension by injection of hydroxyl ions below the surface of the vigorously agitated suspension and separating, hydrothermally treating, if required, drying, calcinating, if required, and reducing the loaded carrier, or

e) introducing into the suspension of the thermally stable oxidic carrier a nickel salt solution under the surface of the suspension while keeping the pH value of the suspension between 4 and 7, separating, drying, calcinating and reducing the loaded carrier.

The method referred to under c) hereinabove is known as such from DE—C—17 67 202. In this way highly suitable catalysts can be obtained. It has also been stated in DE—C—17 67 202 that nickel catalysts thus prepared can be used for methanation of the small quantities of carbon dioxide and carbon monoxide which are present in the gas for the synthesis of ammonia. This relates to the methanation of tiny quantities of carbon monoxide and carbon dioxide (about 0.1% by vol.) present in a hydrogen/nitrogen mixture. It cannot be inferred, however, from said reference, that these catalysts would be suitable for the production of methane from gas mixtures obtained by coal gasification.

It should be noted here that in EU—A—0028835 a two stage process for the production of methane has been described, in the first stage of which a mixture containing 25 to 90% by vol. of hydrogen as well as carbon monoxide and carbon dioxide is passed over a nickel catalyst at a temperature of from 250°C to 550°C to produce a methane rich gas product. For this process the nickel catalyst described in DE—C—17 67 202 may be used. However, the starting gas mixture is obtained by subjecting a gas resulting from coal gasification to the carbon monoxide shift reaction and optionally to a carbon dioxide removal step. Generally the gas mixture contains 5 to 25% by vol. of carbon monoxide, the $H_2/CO$ ratio being in practice 2.5 to 5. It has not been recognized in EU—A—0028835 that gas mixtures having a relatively low hydrogen content and $H_2/CO$ ratio and a relatively high CO content could be converted in one step (therefore without a separate carbon monoxide shift reaction step) into a gaseous product containing predominantly methane, thus making possible an economic production of methane from coal as outlined in the above without being confronted with carbon deposits on the catalyst and in the reactor.

As a matter of fact the above methods of preparing nickel catalysts have been mentioned by way of illustration. Other nickel catalysts meeting the above description can also be used in the process according to the present invention. In particular it has been found that $Ni/SiO_2$ catalysts are highly suitable for the conversion of carbon monoxide with hydrogen and with steam if part of the nickel is still present in the form of Ni(II) ions.

Several nickel catalysts are known, which have been prepared in a very specific way, but which appeared not to be suitable for the process of the invention, although their use for the methanation of gas mixtures containing carbon monoxide has been described. Reference may be made to FR—A—1,074,037. According to this reference an aqueous solution of nickel salt and magnesium salt is introduced into a boiling solution of alkali metal carbonate in such an amount that the pH-value of the solution obtained after precipitation is between 8 and 11. Thereafter kieselguhr or an analogous carrier is added to the resulting suspension, which is agitated until all carbon dioxide is set free. Finally the precipitated mass is thoroughly washed with water. According to NL—A—71 17 619 a nickel catalyst is precipitated in an aqueous suspension formed by an aqueous solution of the nickel salt, an aqueous solution of the alkali metal carbonate and a carrier material, such as silica, which suspension is maintained during the complete or substantially complete precipitation at a temperature between 75°C and 95°C and at a pH-value of 8.0 to 10. When using the s method of preparation of nickel catalysts as described in FR—A—1,074,037 and NL—A—71 17 619 nickel catalysts are obtained in which there is no sufficient chemical bond between the nickel particles and the carrier, so that the catalysts are not suitable for use in the process of the invention.

In one of the above mentioned methods employed to achieve a strong interaction between the nickel particles and the carrier material in the catalysts used in the process of the invention use was made of a carrier, which is to be partially reduced. The compounds or metal atoms produced in reducing the carrier show strong interaction with both the nickel particles and the carrier. An example of such a carrier is $TiO_2$. When heated in a reducing gas $TiO_2$ is reduced to a $TiO_x$ oxide wherein x has a value below 2. The oxide reduced at the interface of the nickel particles ensures a strong bond of the nickel particles to the carrier, provided the nickel has been deposited homogeneously over the carrier and in intimate contact with it. When catalysts are prepared as described in FR—A—2,347,326 by impregnation of nickel metal upon the carrier, drying and subsequent heat treatment of the loaded carrier, the active material is inhomogeneously distributed over the carrier. Especially at more elevated metal loadings, this leads to large nickel clusters, which interact only to a limited extent with the carrier. The interaction of these "loose" particles with the carrier remains small even after partial reduction of the carrier. Therefore catalysts prepared according to FR—A—2,347,326 are not suitable for use in the process of the invention.

In the nickel catalysts used in the process of the invention the presence of an interfacial layer containing nickel ions can be established in several ways. The oxidation state of nickel in catalysts of this type is normally II. According to one method first the total nickel content of the catalyst is determined, e.g. by means of atomic absorption spectrometry. Next the amount of metallic nickel per unit weight of catalyst can be found by measuring the saturation magnetisation of the catalyst sample. The saturation magnetisation found is a direct measure of the amount of metallic nickel present. The difference between the total amount of nickel and the amount of metallic nickel represents the amount of nickel that is present in the interfacial layer in the form of Ni (II) ions.

Alternatively the nickel content that is present as nickel ions in the interfacial layer can be established by measuring the consumption of hydrogen during reduction of the catalyst in a $H_2$ containing atmosphere at temperatures that are also used during the catalyst operation. Subsequently the consumption measured as compared with the volume of hydrogen that would be required for complete reduction of all nickel ions present. From the difference between the two values found the amount of Ni (II) ions can be calculated.

A third way to establish the presence of Ni (II) ions in the active catalyst is to measure the increase in

weight of a catalyst sample, that previously has been reduced under reaction conditions, during reoxidation of the sample in a $O_2$-containing atmosphere. When after reduction part of the nickel is present in the form of Ni (II) ions, the uptake of $O_2$ and hence the increase in weight will be smaller during reoxidation than when after reduction all nickel has been reduced. Comparison of the actual increase in weight with the theoretical increase calculated from the total amount of nickel in the sample yields information on the amount of nickel that is present as Ni (II) ions in the interfacial layer in the active catalyst under reaction conditions.

Preferably catalysts used in the process of the invention contain nickel ions in the interfacial layer in an amount of at least 5%, preferably at least 10%, more preferably at least 20% by weight of the total amount of nickel in the catalyst.

When the catalysts have been prepared as indicated above using $SiO_2$ as a carrier a homogeneous distribution of the nickel over the support is achieved. Hence a strong bonding occurs between the highly dispersed nickel particles and the partially reduced carrier, even at high nickel loadings. The presence of a partially reduced carrier under conditions of the reaction can be established in the active catalyst in two ways. According to one method the metallic nickel content of the catalyst is determined by measuring the saturation magnetisation of a sample of known weight, which previously has been reduced under reaction conditions. Afterwards the sample is reoxidised in an $O_2$-containing atmosphere and simultaneously the increase in weight owing to oxidation of the metallic nickel and, if partially reduced, of the carrier is measured. By comparing the actual increase in weight with the increase that would be expected from oxidation of the metallic nickel only, the extent of reduction of the support can be calculated. In the catalysts used in the process of the invention the increase in weight during oxidation should exceed the increase exclusively caused by oxidation of the nickel metal by at least 10%, preferably by at least 20%.

Alternatively, the extent of reduction of the carrier can be determined by measuring the consumption of $H_2$ during reduction of the completely re-oxidised catalyst in a $H_2$-containing atmosphere at temperatures that are also used in the process of this invention. Subsequently, the consumption measured as compared with the amount of $H_2$ that would be required to yield the amount of nickel which eventually is present as metallic nickel particles in the reduced catalyst. The amount of metallic nickel in the catalyst can be calculated, as indicated above, from a measurement of the saturation magnetisation. From the difference between the actually measured $H_2$-volume and the volume exclusively required for reduction of the nickel in the oxidised form the extent of reduction of the carrier can be determined.

In the catalysts used in the process of the invention the $H_2$ consumption during reduction of the catalyst should exceed the consumption exclusively required for reduction of the oxidic nickel by at least 10%, preferably by at least 20%.

The relative amount of particles, bonded to the carrier through an interfacial layer can be determined in the following three ways:

1. A sample of the reduced active catalyst is investigated in the electron microscope in order to count the number of metallic nickel particles per gram of catalyst as well as their average size. A similar sample is treated with pure CO at ambient or higher pressure at a temperature between 60 and 100°C for such a time period as is required to remove all metallic nickel from the catalyst by formation of gaseous $Ni(CO)_4$. Thus only the nickel that is present as ionic nickel in the interfacial layers remains in the catalyst. By electron microscopic investigation the number of interfacial layers—of roughly the same surface as the original metallic particles — can be counted per gram of catalyst. For a catalyst according to the invention, this number should essentially equal the number, found for the number of metallic nickel particles per gram of catalyst. From the differences between the number of nickel metal particles and the number of nickel interfaces, the amount of "free" of "loose" nickel particles and the weight quantity of "loose" metallic nickel can be calculated.

2. A sample of the reduced active catalyst is treated with a mixture of 90% by volume of $N_2$ and 10% by volume of CO at ambient pressure at a temperature of 400°C for a time period of between 10 and 60 hours. The sample is then cooled down and passivated. Electron microscopic investigation will then show, depending on whether the catalyst is suitable according to the invention or not, that carbon whiskers have been formed. These carbon whiskers are only formed at "loose" metallic nickel particles which are not bonded to the carrier. Thus, the amount of these unbonded particles per gram of catalyst material can be found.

3. In case the carrier material consists of an oxide of a base element which is partially reducible, the unbonded nickel particles can be quantitatively determined by currently available electron microscopic methods.

It has been found that only a small amount of non-chemically bonded nickel can be allowed in the catalysts used in this invention. This amount should not exceed 50 mg, more preferably 30 mg and most preferably 10 mg per $cm^3$ of the catalyst bed, as pointed out already above. When the amount of unbonded nickel exceeds such amounts, the catalyst bed will gradually be plugged due to the growth of carbon whiskers, which already have been discussed above. It was established, that very large nickel particles of dimensions of about 100 nm are slowly deactivated under reaction conditions. Nickel particles of such a large size often contain grain boundaries. It is known (Carbon (1972), pages 601 to 611) that at these grain boundaries carbon can be deposited due to which the particles are broken up. The growth of carbon whiskers continues after the nickel particles have been fragmented, which eventually results in reactor

plugging. However, nickel particles of dimensions smaller than 30 nm generally do not contain grain boundaries. Hence, whisker growth induced by grain boundaries will not occur in these particles. Therefore the catalyst should preferably contain less than 10% by volume of nickel particles larger than 30 nm.

The nickel particle size distribution can be determined by electron microscope study. Another, faster and more accurate method of determining the particle size distribution is by means of magnetism.

Magnetization is measured as a function of the field strength and the particle size distribution is so computed that the experimental magnetization curve is reproduced. It appears that the computed curve produces an unambiguous particle size distribution, corresponding with the distribution found by means of electron microscope examination.

When carbon is intentionally deposited on a catalyst used according to the present invention by passing pure CO over the catalyst at 275°C, and the carbon is then removed by heating the catalyst in a hydrogen stream at temperatures up to 450°C, it appears that the nickel particle size distribution has not significantly changed. This proves that no carbon has been deposited between the nickel particles and the carrier. On the contrary it has been found that in regenerating conventional catalysts in which carbon has been deposited between the nickel particles and the carrier, far bigger nickel particles are formed than the particles that were present in the original catalyst.

When using the nickel catalysts of the invention methane can be produced from gas mixtures with a wide variety of carbon monoxide contents. For instance, gas mixtures can be used which contain far less hydrogen than the amount needed for the classical methanation reaction:

$$CO+3H_2 \rightleftharpoons CH_4+H_2O.$$

Conversion of gas mixtures with a high CO content proved to be possible. This is quite surprising, as it is generally known from the literature referred to above that so far catalyst deactivation by carbon deposition has always occurred in converting gas mixtures with a relatively high carbon monoxide content by means of nickel catalysts.

Moreover, it was found that in using the nickel catalysts according to the present invention, carbon monoxide can be quantitatively converted with steam into methane and carbon dioxide in the absence of hydrogen. Such a reaction has been hitherto unknown. If gas mixtures are used which contain carbon monoxide, hydrogen and water, the carbon monoxide is converted almost quantitatively into methane by use of the nickel catalysts according to the present invention.

An additional advantage of the nickel catalysts used according to the present invention is their low onset temperature. For example, a gas mixture of 50% by vol. of hydrogen and 50% by vol. of carbon monoxide was quantitatively converted into methane and carbon dioxide at 230°C using a nickel catalyst according to the present invention. A low onset temperature of the catalyst is of major importance in performing the present reactions since they are all highly exothermic. The exothermic reactions generally produce a rapid and sharp temperature rise in the reactors. If the reaction starts at a relatively low temperature, the maximum temperature in the reactor can also be kept at a lower temperature. The drawbacks of elevated temperatures are the necessity of adapting the reactor construction, the high demands made on the thermal stability of the catalysts and the less favourable thermodynamic equilibrium position of the above reaction.

Though in the process according to the present invention the quantity of heat released per reacting molecule of carbon monoxide is smaller than in the usual methanation process, the temperature of the reaction mixture may in certain cases rise to an unacceptable level. This applies to mixtures which apart from carbon monoxide contain either hydrogen or steam, or both hydrogen and steam.

In such cases the gas mixture containing carbon monoxide can be diluted with carbon dioxide before it is passed over the nickel catalyst.

Part of the heat of reaction released is then absorbed by the carbon dioxide. Together with the carbon dioxide formed by reaction the diluting carbon dioxide is separated out after the reaction by dissolving it under pressure in a liquid. For this kind of carbon dioxide removal technologically advanced and reliable processes are available. After separation of the carbon dioxide from the gas by means of the liquid the carbon dioxide is removed from the liquid by reducing the pressure and supplying some thermal energy. Part of the carbon dioxide recovered can be recirculated to the initial gas mixture, thus eliminating the need to produce carbon dioxide separately as a diluent. Carbon dioxide supply does not entail incorporation of an additional separating unit either, since the carbon dioxide produced by the reaction should be removed anyway. The presence of carbon dioxide proves to be no detriment to the activity of the catalyst.

The initial gas mixture can also be diluted with methane to absorb part of the heat produced.

Apart from the molar CO-concentration in the gas, the molar $H_2$/CO-ratio is at least equally important for the process according to the invention. Whereas the "classical" methanation reaction requires an $H_2$/CO ratio of at least 3 for proper process operation, in the process according to the invention the molar $H_2$/CO-ratio can be lower than 2.45. We have found that when using the specially defined catalysts according to the invention, an $H_2$/CO ratio below 1.15 will also give very good conversion results. Here, the advantages of the invention even become more pronounced than with higher $H_2$/CO ratios because of the more severe circumstances. Catalysts used until now will rapidly deactivate by deposition of carbon as has been shown in experiments. In addition, we have found it to be quite surprising, that even with extremely

low $H_2/CO$ ratios, i.e. below 0.4, 0.3, 0.2 or even 0.1, the catalysts, in which metallic nickel particles are chemically bonded to an oxidic carrier, give good conversion results. If the $H_2/CO$ ratio of the gas mixture is about 0.9 to 1.1 the initial gas mixture should preferably be mixed with a quantity of steam corresponding to at least 10 mol % related to the carbon monoxide present. In using gas mixtures with $H_2/CO$ ratios below 0.9 it proved to be desirable to add more steam. It was found that the quantity of steam required for a satisfactory performance of the process is at least 50 mol % related to the difference between the number of moles of carbon monoxide and the number of moles of hydrogen. This can be expressed in the following formula:

$$\text{moles } H_2O=[(0.5 \text{ to } 0.6)\times\text{moles CO}] \text{ minus } [0.5\times\text{moles } H_2].$$

The temperatures at which the conversions can be carried out range from 230°C to 700°C. More specifically these temperatures lie in the range of from 230°C to 600°C and advantageously from 230°C to 550°C. The pressures at which the conversions can be carried out range from 100 to 10,000 kPa, more specifically from 500 to 8,000 kPa and advantageously from 1,500 to 4,500 kPa. Before according to the process of this invention the gas mixture is passed over the catalyst, it is substantially freed of sulphur compounds. If in removing sulphur compounds from the gas mixture steam condenses it need not be supplemented as for methanation little or no steam is required. Further, the $CO_2$-content of the mixture entering the desulphurization plant is lower than in the case where first a shift reaction has been performed.

The process according to the invention can, for instance, be carried out with excellent results in an apparatus as described in US—A—4,133,825. This use is only mentioned as an example, however, because it has been found that in using the nickel catlaysts according to the present invention all gas mixtures can be handled which contain more than 5% by vol. of carbon monoxide. In general the carbon monoxide content of the gas mixtures to be treated will be from 5—80% by vol. As explained above, however, the catalysts are especially useful for the production of methane from raw gas mixtures with relatively high carbon monoxide contents, at least more than 10—20% and preferably more than 25% by vol.

The process according to the invention can also be carried out in a fluidized bed. In the already mentioned paper by W. Lommerzheim and K. Hedden (1st Gas Research Conference, Chicago, page 49) it was noted that carbon deposition leads to a decreased mechanical strength of the catalyst. Since with a catalyst according to the invention, abrasion due to carbon deposition is avoided, it is very favourable to use such a catalyst in a fluidized-bed process aimed at the conversion of high-CO, low $H_2/CO$-ratio feed gas to form methane (and carbondioxide).

The following examples are given as a further illustration of the invention but do not limit its scope. Example I describes the preparation of the nickel catalyst which is used in Examples II to VIII for the production of methane from gas mixtures.

Example I

43.6 g $Ni(NO_3)_2 . 6H_2O$ was dissolved in 1 litre of water in which 9 g silica (trade mark Aerosil of Degussa) having a surface area of 380 $m^2/g$ had been suspended. The temperature of the vigorously agitated suspension was then raised to 90°C and the solution acidified dropwise with nitric acid to a pH of 2.

Subsequently 27.0 g of urea was added, as a result of which the pH value gradually rose and nickel was deposited on the silica. The loaded carrier was then separated from the liquid, dried at 120°C, pressed and cut into pieces of sizes ranging from 1.5 to 2.5 mm.

Afterwards the pieces were dehydrated in a nitrogen stream at a temperature up to 450°C and reduced in a stream of 10% hydrogen in argon at 450°C for at least 80 hours. Before the tests described in Example II to VIII were carried out the catalyst was freed from adsorbed hydrogen in a nitrogen stream at 450°C for 2 hours. The nickel particle size distribution of the catalyst thus obtained is shown in Table A.

TABLE A

| Fraction, % by vol. | Diameter, nm |
|---|---|
| 0 | <2.5 |
| 3.40 | 2.69 |
| 5.30 | 2.90 |
| 2.89 | 3.13 |
| 7.17 | 3.37 |
| 10.67 | 3.63 |
| 6.44 | 3.91 |
| 9.12 | 4.22 |
| 3.20 | 4.54 |
| 1.60 | 4.90 |
| 7.33 | 5.27 |
| 4.28 | 5.68 |
| 8.43 | 6.12 |
| 5.68 | 6.60 |
| 7.15 | 7.11 |
| 5.10 | 7.66 |
| 3.61 | 8.26 |
| 3.13 | 8.90 |
| 0.00 | 9.58 |
| 0.00 | 10.3 |
| 0.00 | 11.1 |
| 4.22 | 12.0 |
| 1.28 | 12.9 |
| 0 | >13 |

After this catalyst had been used in three tests, in which carbon from carbon monoxide was deposited at 275°C and subsequently removed again with hydrogen at temperatures rising to 450°C, the nickel particle size distribution was again determined. The result is shown in Table B.

TABLE B

| Fraction, % by vol. | Diameter, nm |
|---|---|
| 0 | <3 |
| 7.29 | 3.04 |
| 6.05 | 3.31 |
| 6.73 | 3.60 |
| 6.95 | 3.92 |
| 4.59 | 4.26 |
| 5.57 | 4.63 |
| 4.98 | 5.04 |
| 0.0 | 5.48 |
| 2.97 | 5.96 |
| 5.07 | 6.48 |
| 9.12 | 7.05 |
| 8.37 | 7.66 |
| 8.03 | 8.33 |
| 7.22 | 9.06 |
| 5.94 | 9.86 |
| 4.50 | 10.7 |
| 3.15 | 11.7 |
| 3.47 | 12.7 |
| 0 | >13 |

Example II

Two series of experiments were carried out on laboratory scale using the catalyst prepared according to Example I. In the A series a catalyst bed with a length of 2.5 cm was filled with a catalyst containing 1.15 g of nickel in total. For the tests of the A series the carbon monoxide and the hydrogen were diluted with nitrogen.

The nitrogen was found to have no effect on the catalyst activity. In test 1 of the A series a mixture of hydrogen and carbon monoxide ($H_2/CO=1.3$) was passed through the reactor at a temperature of 285°C.

After about 1 hour a stationary condition was reached in which the carbon monoxide was fully converted. After termination of the test, i.e. after 48 hours, no reduction of catalyst activity was observed.

In various tests of the A series the gas mixture was saturated with water by passing it through a saturator at a rate of 50 ml/minute, before the gas mixture was passed over the catalyst. In the saturator the gas bubbled through a thermostatically controlled water reservoir with 18 cm head of water.

In test 2 of the A series the conversion at 280°C of carbon monoxide with steam, i.e. without hydrogen, was examined. In the stationary condition a mixture of $CO_2$ and $CH_4$ was obtained with a $CO_2/CH_4$ ratio of 3. In this test, too, complete conversion of the carbon monoxide and no deactivation was found, nor was deactivation observed in any of the other tests of the A series.

Tests 5, 6a and 7 of the A series showed that under virtually identical conditions the conversion of carbon monoxide and hydrogen decreased at a rise of temperature. Tests 5, 6a and 7 of the A series also showed that under virtually identical conditions the $CO_2/CH_4$ ratio of the product gas increased from 0.8 at 400°C to 1.2 at 500°C. At a temperature of 470°C the conversion of carbon monoxide was found to be raised by increasing the partial water pressure. In the B series a catalyst bed with a length of 15.0 cm was used which had been filled with a catalyst containing 5.4 g of nickel.

The gas mixture used in the B series was not diluted with nitrogen. In spite of the increased partial pressure of the carbon monoxide the catalyst showed no sign of deactivation after 72 hours of continuous operation.

Whereas in the A series the carbon monoxide conversion rapidly decreased at temperatures below 265°C, carbon monoxide conversion in the B series proved still complete at 230°C (B series, test F).

The results of the tests are listed in Table C.

TABLE C

| Test | Reactor temperature (°C) | Reaction components (% by vol. on dry basis) $H_2$ | CO | $N_2$ | Saturator T (°C) | P (kPa) | $CH_4$ | Products (% by vol. on dry basis) $CO_2$ | $H_2$ | CO | $N_2$ | Duration (h) |
|---|---|---|---|---|---|---|---|---|---|---|---|---|
| A series 1 | 285 | 10.7 | 8.1 | 81.2 | — | — | 5.5 | 2.8 | 0.4 | — | 91.3 | 48 |
| 2 | 280 | — | 8.6 | 91.4 | 38 | 6.67 | 2.2 | 6.6 | — | — | 91.2 | 24 |
| 3 | 285 | 9.9 | 18.3 | 71.8 | 41 | 7.73 | 7.9 | 10.9 | 0.7 | — | 80.5 | 40 |
| 4 | 280 | 5.4 | 14.6 | 80.0 | 47 | 10.67 | 5.0 | 8.9 | 0.6 | — | 85.5 | 45 |
| 5 | 400 | 11.2 | 9.9 | 78.9 | 16 | 1.87 | 5.4 | 4.5 | 3.2 | 0.4 | 86.5 | 48 |
| 6a | 470 | 8.5 | 8.5 | 83.0 | 14 | 1.60 | 2.8 | 3.3 | 4.1 | 2.0 | 87.8 | (2) |
| b | 470 | 8.2 | 8.2 | 83.6 | — | — | 3.0 | 2.7 | 2.9 | 2.4 | 89.0 | (18) |
| c | 470 | 8.4 | 8.2 | 83.4 | 30 | 4.27 | 2.8 | 3.9 | 5.2 | 1.5 | 86.6 | (2) |
| d | 470 | 8.4 | 8.1 | 83.5 | 43 | 8.66 | 2.7 | 4.3 | 6.0 | 1.2 | 85.8 | (2) |
| e | 470 | 8.4 | 8.1 | 83.5 | 52 | 13.6 | 2.6 | 4.2 | 6.1 | 1.1 | 86.0 | (2) |
| f | 470 | 8.5 | 6.1 | 85.4 | — | — | 2.8 | 2.0 | 3.4 | 1.6 | 90.2 | 44 (18) |
| 7 | 500 | 9.1 | 8.5 | 82.4 | 16 | 1.87 | 2.5 | 3.1 | 6.3 | 2.7 | 85.4 | 72 |
| B series 1a | 275 | 52.5 | 47.5 | — | 20 | 2.40 | 49.1 | 50.2 | 0.7 | — | — | (18) |
| b | 250 | 52.5 | 47.5 | — | 20 | 2.40 | 50.4 | 49.6 | — | — | — | (2) |
| c | 427 | 52.5 | 47.5 | — | 20 | 2.40 | 44.8 | 47.4 | 7.1 | 0.7 | — | (2) |
| d | 427 | 53.4 | 46.6 | — | 29 | 4.00 | 44.4 | 47.0 | 8.0 | 0.6 | — | (2) |
| e | 427 | 53.7 | 46.3 | — | 35 | 5.60 | 44.3 | 46.0 | 8.7 | 1.0 | — | (2) |
| f | 230 | 52.8 | 47.2 | — | 21 | 2.53 | 49.3 | 49.6 | 1.1 | — | — | 74 (48) |

Example III

In the process according to this example use was made of an apparatus as schematically represented in Figure 1 of the drawings. A mixture of 50% by vol. of hydrogen and 50% by vol. of carbon monoxide was supplied through line 1 at a rate of 44.6 mol/h, a pressure of 2200 kPa and a temperature of 420°C to mixing unit 2, in which it was mixed with a gas mixture consisting of 5.5 mol $H_2$, 1,2 mol CO, 14.3 mol $CH_4$, 16.4 mol $CO_2$ and 0.3 mol $H_2O$ and having a temperature of 71°C. The mixture leaving mixing unit 2 was heated up to 250°C in heater 3 and it was passed in reactor 4 over a catalyst prepared in the way as described in Example I. The mixture leaving the reactor through line 5 consisted of 9.9 mol $H_2$, 16.7 mol CO, 20 mol $CH_4$, 16.5 mol $CO_2$ and 6 mol $H_2O$, and had a temperature of 700°C. In mixing unit 6 this mixture was mixed with 34.9 mol $H_2O$ which were supplied through line 7 at a temperature of 15°C and a pressure of 2500 kPa. The mixture obtained in unit 6, the temperature of which was 250°C, was then passed in reactor 8 over a similar catalyst as used in reactor 4. The product leaving reactor 8 through line 9 at a temperature of 550°C consisted of 9.1 mol $H_2$, 2 mol CO, 23.8 mol $CH_4$, 27.4 mol $CO_2$ and 33.8 mol $H_2O$. In heat exchanger 10 the product was cooled down to 250°C and in cooler 11 it was further cooled with water to 45°C.

In cooling down 33.3 mol water condensed which was drained off through line 12.

The remainder of the product was discharged through line 13, 40% by vol. being carried off through line 14 as product and 60% by vol. being recirculated via line 15, compressor 16 (the pressure being raised from 1520 to 2160 kPa) and line 17 to feed line 1.

The product consisted of 44.5% by vol. of $H_2$, 3.1% by vol. of CO, 37.9% by vol. of $CH_4$, 43.6% by vol. of $CO_2$ and 0.8% by vol. of $H_2O$.

In using the same product but recirculating 70% of the product the final temperature in reactor 4 was 642°C and in reactor 8 485°C. In this case 38.9 mol water were supplied through line 7 and 38 mol water were drained off through line 12.

The composition of the final product was then 8.1% by vol. of $H_2$, 1.3% by vol. of CO, 43,2% by vol. of $CH_4$, 46,6% by vol. of $CO_2$ and 0.9% by vol. of $H_2O$.

The term "mol" used in this example is not to be understood as an absolute value; it is just an indication of the relative quantities of the components.

Example IV

A cylindrical reactor tube made of stainless steel was filled with a sample of the catalyst prepared and pretreated as described in Example I. The catalyst bed was 1.8 cm in diameter and 17 cm in height. The catalyst sample in the reactor contained 6.3 g of reduced nickel. A gas mixture comprising 15% by volume of $H_2$, 10% by volume of CO and 75% by volume of $N_2$ was passed through the catalyst bed at a space velocity of 1500 $hr^{-1}$ and a total pressure of 600 kPa. During the experiment the temperature of the catalyst bed was measured continuously at nine heights in the bed. The temperature of the reaction mixture at the entrance of the bed was 250°C, while the temperature at the exit of the poorly isolated laboratory reactor was 230°C. To establish whether catalyst plugging (owing to carbon deposition) occurred the pressure drop over the catalyst bed was recorded continuously. The experiment was continued over a prolonged period of about 1000 hr. The product gas, the composition of which did not change throughout the experiment, consisted of 6.9% by volume of $CH_4$, 4.1% by volume of $CO_2$, 0.2% by volume of CO, 0.0% $H_2$, $H_2O$ (not determined quantitatively) and $N_2$. At the end of the experiment catalyst activity had not decreased and no increase in pressure drop had been observed. The pressure drop being at a constant low level indicated that severe carbon deposition and catalyst plugging had not occurred.

By way of comparison the experiment described hereinbefore was repeated, but now with a commercially available methanation catalyst, Harshaw Ni 6458. The reactor was loaded with 41.4 g of this catalyst, which was pretreated according to prescriptions of the manufacturer. Subsequently, the reaction mixture consisting of 15% by volume of $H_2$, 10% by volume of CO and 75% by volume of $N_2$ was passed through the catalyst bed at a space velocity of 1500 $hr^{-1}$ and a total pressure of 600 kPa. The temperature of the gas mixture at the entrance of the bed was 250°C, whilst the temperature at the reactor outlet was 230°C. Initially the catalyst displayed an activity which was similar to that observed in the experiment described hereinbefore: the product gas was composed of 6.9% by volume of $CH_4$, 4.1% by volume of $CO_2$, 0.2% by volume of CO, 0.0% $H_2$, $H_2O$ (not determined quantitatively) and $N_2$. However, after about 75 hr the pressure drop over the catalyst bed started to increase appreciably. Fig. 2 of the drawings shows a plot of the pressure drop versus time. After about 225 hr the deposition of carbon, which brought about the rise in pressure drop over the reactor, had increased so much that the experiment had to be stopped because the catalyst bed got plugged. For comparison Fig. 2 also shows the constant small pressure drop observed during the experiment described hereinbefore, in which under similar conditions a catalyst according to the invention was used.

Example V

The reactor used in Example IV was filled with the catalyst prepared and pretreated as described in Example I. The catalyst sample contained 6.1 g of reduced nickel. A reaction mixture containing 26.9% by volume of $H_2$, 17.9% by volume of CO and 55.2% by volume of $N_2$ was passed through the catalyst bed at a space velocity of 1500 $hr^{-1}$ and a total pressure of 600 kPa. The temperature of the gas at the entrance of the bed was 260°C, whereas the temperature at the reactor exit was 190°C. The product gas composed of 14.0%

by volume of $CH_4$, 8.2% by volume of $CO_2$, 0.2% by volume of CO, 0.0% $H_2$, $H_2O$ (not determined quantitatively) and $N_2$. The product gas composition did not change during the experiment, which lasted for 650 hr. After 650 hr the experiment was stopped. At that time the pressure drop over the catalyst bed was negligibly small, indicating that carbon deposition and catalyst plugging had not occurred.

Example VI

The equipment described in Example IV was used. The reactor was filled with a sample of the catalyst prepared and pretreated as indicated in Example 1. The catalyst sample contained 5.5 g of reduced nickel. Before a reaction mixture comprising 23,5% by volume of CO, 11.8% by volume of $H_2$ and 64,7% by volume of $N_2$ entered the reactor, an amount of 49 mmol $min^{-1}$ of water was added to the mixture in the preheater kept at 450°C. The temperature of the reactants including the evaporated water at the entrance of the reactor was 350°C. The products left the reactor at 370°C. The space velocity in the reactor was 1500 $hr^{-1}$ at a total pressure of 600 kPa. The product gas composition, which was constant over the entire duration of the experiment of 500 hr, was determined to contain 6.5% by volume of $CH_4$, 13.8% by volume of $CO_2$, 6.0% by volume of $H_2$, 1.0% by volume of CO, $H_2O$ (not determined quantitatively) and $N_2$. After 500 hr the pressure drop over the catalyst bed had not increased, which implies the absence of catalyst plugging owing to severe carbon deposition.

Example VII

The reactor described in Example IV was filled with the catalyst prepared and pretreated according to the procedures of Example I. The catalyst sample contained 5.2 g of reduced nickel. Before a mixture of 11.2% by volume of CO and 88.8% by volume of $N_2$ was led into the reactor an amount of 29 mmol $min^{-1}$ of water was added to it in the preheater kept at 450°C. After evaporation of the water the gas mixture entered the reactor at a temperature of 350°C. At the exit of the reactor the temperature was 386°C. In the reactor the space velocity was 1500 $hr^{-1}$ and the total pressure 600 kPa. The reaction that proceeded in the catalyst bed led to a product containing 2.0% by volume of $CH_4$, 7.9% by volume of $CO_2$, 0.1% by volume of CO, 1.0% by volume of $H_2$, $H_2O$ (not determined quantitatively) and $N_2$. The product composition remained constant during the experiment which lasted for 1000 hr. After 1000 hr the pressure drop over the reactor was still as small as at the beginning of the experiment. The permanent low level of the pressure drop reveals the absence of catalyst plugging owing to carbon deposition.

Example VIII

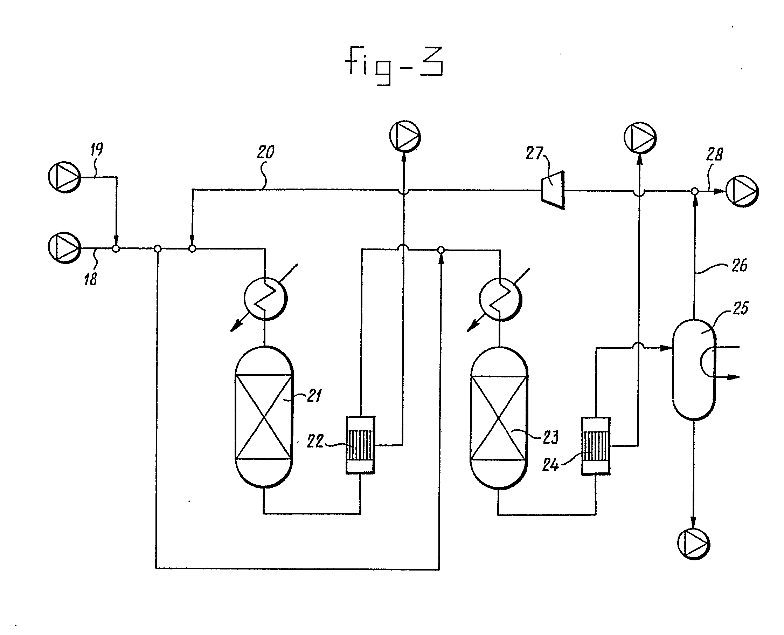

A process according to the invention was carried out in an apparatus shown schematically in Fig. 3 of the drawings.

A desulphurized, carbonmonoxide rich gas supplied through line 18 was mixed with steam supplied through line 19 and the mixture was combined with recycled product gas supplied through line 20. The resulting mixture was fed to reactor 21 filled with a catalyst according to the invention. In this reactor the gas was partly converted. The gas leaving this reactor was cooled down through a waste heat boiler 22 and then fed to a second reactor 23 filled with the same catalyst, in which reactor the gas was further converted. The gas leaving reactor 23 was again cooled down through a waste heat boiler 24. Part of the gas was recycled to the first reactor 21 through cooler 25, line 26, compressor 27 and line 20. The product gas left the system through line 28.

In Table D are given the product gas composition for a case where the entrance temperatures of both shift-methanation reactors are 350°C and the recycle ratio is 3:1 (i.e. 75% of the gas leaving the second reactor is recycled to the first reactor). After the removal of carbondioxide the gas composition as mentioned in Table D has a Wobbe-index of 45.5 $MJ/m^3$, being of the same order as the Wobbe-index of the natural gas produced in the province of Groningen in the Netherlands.

TABLE D

| Component % by vol. | Feed gas 1) | Product gas 2) | Purified product gas 3) |
|---|---|---|---|
| $H_2$ | 25.8 | 3.2 | 7.9 |
| CO | 25.1 | 1.6 | 3.9 |
| $CH_4$ | 4.6 | 34.8 | 86.0 |
| $CO_2$ | 16.6 | 59.8 | 2.2 |
| $H_2O$ | 27.9 | 0.6 | 0 |
| T (°C) | 350 | 464 | 30 |

Notes
1) entering reactor 21.
2) leaving reactor 23.
3) after $CO_2$-removal.

**Claims**

1. A process for the preparation of methane from gas mixtures containing hydrogen and/or steam and at least 5% by volume of carbonmonoxide by means of a nickel catalyst, which contains metallic nickel particles as an active catalyst component on a thermally stable oxidic carrier, characterized in that a gas mixture, in which the molar ratio $H_2/CO$ is below 2.45, is contacted with a nickel catalyst, in which the metallic nickel particles are chemically bonded to the thermally stable oxidic carrier.

2. A process as claimed in claim 1, characterized in that the chemical bond between the metallic nickel particles and the oxidic carrier is provided by an interfacial layer different from the oxidic carrier, which layer consists of a compound containing oxygen and at least one member selected from the group consisting of nickel and the carrier base element or elements.

3. A process as claimed in claim 2, characterized in that the interfacial layer consists of
a) a compound containing nickel ions, or
b) a stable non-stoichiometric oxide of the carrier base element or elements, or
c) a compound containing nickel and the carrier base element or elements.

4. A process as claimed in claim 2, characterized in that the carrier is an oxide of a carrier base element or elements, which form(s) only stoichiometric oxides, and the interfacial layer contains nickel ions.

5. A process as claimed in claim 4, characterized in that the carrier is an oxide of at least one member of the group consisting of Si, Al and Mg.

6. A process as claimed in claim 2, characterized in that the carrier is an oxide of a carrier base element or elements, which can form a stable non-stoichiometric oxide, and the interfacial layer comprises such non-stoichiometric oxides.

7. A process as claimed in claims 2 to 6, characterized in that the interfacial layer has a thickness between 0.2 and 10 nm, preferably between 0.5 and 5 nm.

8. A process as claimed in claims 1 to 7, characterized in that per $cm^3$ of the catalyst bed there is not more than 0.05 g, preferably not more than 0.03 g, and most preferably not more than 0.01 g metallic nickel, which is not chemically bonded to the carrier.

9. A process as claimed in claims 1 to 8, characterized in that the nickel particle size distribution is such that less than 10% by volume of the particles is smaller than 2 nm and less than 10% by volume of the particles is larger than 30 nm.

10. A process as claimed in claims 1 to 9, characterized in that the nickel catalyst is obtainable by
a) mixing an aqueous solution of a nickel salt and an aqueous solution of a salt of the base element or elements, the oxide of which is the thermally stable oxidic carrier, raising the pH-value up to a level where the dissolved nickel and base element or elements ions have been substantially completely precipitated, ageing, if required, the precipitate in the solution, separating, hydrothermally treating, if required, and drying, calcinating and reducing the precipitate,
b) mixing an aqueous solution of a nickel salt and an aqueous solution of a salt of the base element or elements, the oxide of which is the thermally oxidic carrier, with an aqueous solution of an oxalate or formate, separating, drying, calcinating and reducing the precipitate, or
c) suspending in a finely divided form the thermally stable oxidic carrier in a dilute aqueous solution of a nickel salt and precipitating a nickel compound at an elevated temperature and with vigorous agitation by forming hydroxyl ions by a chemical reaction, which is known *per se*, of compounds contained also in the solution, not faster than this is the case by hydrolysis of an aqueous solution of urea, which is present in the

solution in an amount of 1 to 10 times the amount stoichiometrically required, followed by separating, calcinating and reducing the loaded carrier, or

d) suspending in a finely divided form the thermally stable oxidic carrier in a dilute aqueous solution of a nickel salt, raising the pH-value of the resulting suspension by injection of hydroxyl ions below the surface of the vigorously agitated suspension and separating, hydrothermally treating, if required, drying, calcinating, if required, and reducing the loaded carrier, or

e) introducing into the suspension of the thermally stable oxidic carrier a nickel salt solution under the surface of the suspension while keeping the pH-value of the suspension between 4 and 7, separating, drying, calcinating and reducing the loaded carrier.

11. A process as claimed in claims 1 to 10, characterized in that the molar ratio $H_2/CO$ is below 1.15.

12. A process as claimed in claim 11, characterized in that the feed gas has a molar $H_2/CO$ ratio below 1 and contains steam in an amount complying with the following formula:

$$\text{moles } H_2O=[(0.5 \text{ to } 0.6)\times\text{moles CO}] \text{ minus } [0.5\times\text{moles } H_2]$$

**Patentansprüche**

1. Verfahren zur Herstellung von Methan aus Gasmischungen, die Wasserstoff und/oder Dampf und mindestens 5 Vol.-% Kohlenmonoxyd enthalten, mittels eines Nickelkatalysators, der metallische Nickelpartikel als aktive katalytische Komponente auf einem thermisch stabilen, oxydischen Trägerstoff enthält, dadurch gekennzeichnet, daß eine Gasmischung, in der das Molverhältnis $H_2/CO$ kleiner als 2,45 ist, mit einem Nickelkatalysator in Kontakt gebracht wird, in dem die metallischen Nickelpartikel chemisch an den thermisch stabilen, oxydischen Trägerstoff gebunden sind.

2. Verfahren nach Anspruch 1, dadurch gekennzeichnet, daß die chemische Bindung zwischen den metallischen Nickelpartikeln und dem oxydischen Trägerstoff durch eine Grenzflächenschicht geschaffen ist, die von dem oxydischen Trägerstoff verschieden ist, wobei die Schicht aus einer Verbindung besteht, die Sauerstoff und mindestens ein Element enthält, das aus der Gruppe ausgewählt ist, die aus Nickel und dem Trägerstoffgrundelement oder den Trägerstoffgrundelementen besteht.

3. Verfahren nach Anspruch 2, dadurch gekennzeichnet, daß die Grenzflächenschicht besteht aus

a) einer Verbindung, die Nickelionen enthält, oder

b) einem stabilen nicht-stöchiometrischen Oxid des Trägerstoffgrundelementes oder der Trägerstoffgrundelemente, oder

c) einer Verbindung, die Nickel und das Trägerstoffgrundelement oder die Trägerstoffgrundelemente enthält.

4. Verfahren nach Anspruch 2, dadurch gekennzeichnet, dass der Trägerstoff ein Oxid des Trägerstoffgrundelementes oder der Trägerstoffgrundelemente ist, welches (welche) nur stöchiometrische Oxide bildet (bilden) und die Grenzflächenschicht Nickelionen enthält.

5. Verfahren nach Anspruch 4, dadurch gekennzeichnet, dass der Trägerstoff ein Oxid von mindestens einem Element aus der Gruppe ist, die aus Si, Al und Mg besteht.

6. Verfharen nach Anspruch 2, dadurch gekennzeichnet, dass der Trägerstoff ein Oxid des Trägerstoffgrundelementes oder der Trägerstoffgrundelemente ist, das ein stabiles nichtstöchiometrisches Oxid bilden kann, und die Grenzflächenschicht diese nicht-stöchiometrischen Oxide umfasst.

7. Verfahren nach Anspruch 2 bis 6, dadurch gekennzeichnet, daß die Grenzflächenschicht eine Dicke zwischen 0,2 und 10 nm, vorzugsweise zwischen 0,5 und 5 nm hat.

8. Verfahren nach Anspruch 1 bis 7, dadurch gekennzeichnet, daß es pro $cm^3$ des Katalysatorbettes nicht mehr als 0,05 g, vorzugsweise nicht mehr als 0,03 g und am bevorzugtesten nicht mehr als 0,01 g metallisches Nickel gibt, das nicht chemisch an den Träger gebunden ist.

9. Verfahren nach Anspruch 1 bis 8, dadurch gekennzeichnet, daß die Größenverteilung der Nickelpartikel so ist, daß weniger als 10 Vol.-% der Partikel geringer als 2 nm sind und weniger als 10 Vol.-% der Partikel größer als 30 nm sind.

10. Verfahren nach Anspruch 1 bis 9, dadurch gekennzeichnet, dass der Nickelkatalysator erhältlich ist durch

a) Mischen einer wässrigen Lösung eines Nickelsalzes und einer wässrigen Lösung eines Salzes des Grundelementes oder der Grundelemente, dessen Oxid der thermisch stabile oxydische Trägerstoff ist, Erhöhen des pH-Wertes bis zu einem Wert, bei dem die gelösten Nickelionen und die gelösten Ionen des Grundelementes oder der Grundelemente im wesentlichen vollständig ausgefällt sind, Altern, falls erforderlich, des Niederschlages in der Lösung, Abtrennen, hydrothermale Behandlung, falls erforderlich, und Trocknen, Kalzinieren und Reduzieren des Niederschlages,

b) Mischen einer wässrigen Lösung eines Nickelsalzes und einer wässrigen Lösung eines Salzes des Grundelementes oder der Grundelemente, dessen Oxid der thermische oxydische Trägerstoff ist, mit einer wässrigen Lösung eines Oxalats oder Formiats, Abtrennen, Trocknen, Kalzinieren und Reduzieren des Niederschlages, oder

c) Suspendieren des thermisch stabilien oxydischen Trägerstoffs in fein verteilter Form in einer verdünnten wässrigen Lösung eines Nickelsalzes und Ausfällen einer Nickelverbindung bei einer erhöhten

Temperatur und mit kräftigem Rühren durch Formen von Hydroxylionen durch eine chemische Reaktion, die an sich bekannt ist, von Verbdingungen, die auch in der Lösung enthalten sind, nicht schneller als es der Fall ist durch Hydrolyse einer wässrigen Lösung von Harnstoff, der in der Lösung in einer Menge des 1- bis 10-fachen der Menge vorhanden ist, die stöchometrisch erforderlich ist, gefolgt von Abtrennen, Kalzinieren und Reduzieren des beladenen Trägerstoffes, oder

d) Suspendieren des thermisch stabilen oxydischen Trägerstoffes in fein verteilter Form in einer verdünnten, wässrigen Lösung eines Nickelsalzes, Erhöhung des pH-Wertes der entstehenden Suspension durch Injektion von Hydroxylionen unter die Oberfläche der Kräftig gerührten Suspension und Abtrennen, hydro-thermale Behandlung, falls erforderlich, Trocknen, Kalzinieren, falls erforderlich, und Reduzieren des beladenen Trägerstoffes, oder

e) Einlassen einer Nickelsalzlösung in die Suspension der thermisch stabilen oxydischen Trägerstoffes unter die Oberfläche der Suspension, wobei der pH-Wert der Suspension zwischen 4 und 7 gehalten wird, Abtrennen, Trocknen, Kalzinieren und Reduzieren des beladenen Trägerstoffes.

11. Verfahren nach Anspruch 1 bis 10, dadurch gekennzeichnet, daß das Molverhältnis $H_2/CO$ kleiner, als 1,15 ist.

12. Verfahren nach Anspruch 11, dadurch gekennzeichnet, daß die Gascharge ein molares $H_2/CO$-Verhältnis kleiner als 1 hat, und Dampf in einer Menge enthält, die der folgenden Formel entspricht:

$$\text{Mole } H_2O=[(0,5 \text{ bis } 0,6)\times\text{Mole CO}]-[0,5\times\text{Mole } H_2]$$

**Revendications**

1. Procédé de préparation du méthane à partir de mélanges gazeux contenant de l'hydrogène et/ou de la vapeur et au moins 5% en volume d'oxyde de carbone, au moyen d'un catalyseur au nickel qui contient des particules métalliques de nickel comme constituant actif du catalyseur sur un support d'oxyde thermiquement stable, caractérisé en ce qu'un mélange gazeux, dans lequel le rapport molaire $H_2/CO$ est inférieur à 2,45, est mis en contact avec un catalyseur au nickel, dans lequel les particules métalliques de nickel sont liées chimiquement au support d'oxyde thermiquement stable.

2. Procédé suivant la revendication 1, caractérisé en ce que le liaison chimique entre les particules métalliques de nickel et le support d'oxyde est réalisé par une couche interfaciale différente du support d'oxyde, laquelle couche consiste en un composé contenant de l'oxygène et au moins un élément choisi dans le groupe constitué du nickel et de l'élément ou des éléments de base du support.

3. Procédé suivant la revendication 2, caractérisé en ce que la couche interfaciale consiste en

a) un composé contenant des ions nickel, ou

b) un oxyde non stoichiométrique stable de l'élément ou des éléments de base du support, ou

c) un composé contenant du nickel et l'élément ou les éléments de base du support.

4. Procédé suivant la revendication 2, caractérisé en ce que le support est un oxyde d'un élément ou d'éléments de base du support, qui forme(nt) seulement des oxydes stoïchiométriques, et en ce que la couche interfaciale contient des ions nickel.

5. Procédé suivant la revendication 4, caractérisé en ce que le support est un oxyde d'au moins un membre du groupe constitué de Si, Al et Mg.

6. Procédé suivant la revendication 2, caractérisé en ce que le support est un oxyde d'un élément ou ou d'éléments de base du support, qui peuvent former un oxyde non stoïchiométrique stable, et en ce que la couche interfaciale comprend ces oxydes non stoïchiométriques.

7. Procédé suivant les revendications 2 à 6, caractérisé en ce que la couche interfaciale a une épaisseur entre 0,2 et 10 nm, de préférence entre 0,5 et 5 nm.

8. Procédé suivant les revendications 1 à 7, caractérisé en ce que par $cm^3$ de lit de catalyseur, il n'y a pas plus de 0,05 g, de préférence pas plus de 0,03 g, et mieux encore pas plus de 0,01 g de nickel métallique, qui n'est pas chimiquement lié au support.

9. Procédé suivant les revendications 1 à 8, caractérisé en ce que la répartition des tailles de particules de nickel est telle que moins de 10% en volume des particules soient plus petites que 2 nm et que moins de 10% en volume des particules soient plus grandes que 30 nm.

10. Procédé suivant les revendications 1 à 9, caractérisé en ce que le catalyseur au nickel peut être obtenu

a) en mélangeant une solution aqueuse d'un sel de nickel et une solution aqueuse d'un sel de l'élément ou des éléments de base, dont l'oxyde est le support d'oxyde thermiquement stable, en élevant le pH à un niveau où les ions de nickel dissous et d'élément ou d'éléments de base ont été précipités de façon pratiquement complète, en vieillissant, si nécessaire, le précipité dans la solution, en séparant, en soumettant à un traitement hydrothermal, si nécessaire, et en séchant, calcinant et réduisant le précipité,

b) en mélangeant une solution aqueuse d'un sel de nickel et une solution aqueuse d'un sel de l'élément ou des éléments de base, dont l'oxyde est le support d'oxyde thermiquement stable, avec une solution aqueuse d'un oxalate ou formiate, en séparant, séchant, calcinant et réduisant le précipité, ou

c) en mettant en suspension sous forme finement divisée le support d'oxyde thermiquement stable dans une solution aqueuse diluée d'un sel de nickel et en précipitant un composé du nickel à température élevée et en agitant énergiquement, en formant des ions hydroxyl par une réaction chimique, qui est

connue en soi, entre des composés également contenus dans la solution, à une vitesse non supérieure à celle observée dans le cas de l'hydrolyse d'une solution aqueuse d'urée, qui est présente dans la solution dans une quantité 1 à 10 fois supérieure à la quantité stoïchiométriquement nécessaire, puis en séparant, calcinant et réduisant le support chargé, ou

d) en mettant en suspension sous forme finement divisée le support d'oxyde thermiquement stable dans une solution aqueuse diluée d'un sel de nickel, en élevant le pH de la suspension obtenue par injection d'ions hydroxyle au-dessous de la surface de la suspension énergiquement agitée et en séparant, en traitant par voie hydrothermale, si nécessaire, en séchant, calcinant, si nécessaire, et en réduisant le support chargé, ou

e) en introduisant dans la suspension du support d'oxyde thermiquement stable une solution de sel de nickel sous la surface de la suspension tout en maintenant le pH de la suspension en 4 et 7, en séparant, séchant, calcinant et réduisant le support chargé.

11. Procédé suivant les revendications 1 à 10, caractérisé en ce que le rapport molaire $H_2/CO$ est au-dessous de 1,15.

12. Procédé suivant la revendication 11, caractérisé en ce que le gaz d'alimentation a un rapport molaire $H_2/CO$ inférieur à 1 et contient de la vapeur dans une quantité satisfaisante à la formule suivante:

$$\text{moles } H_2O=[(0,5 \text{ à } 0,6)\times\text{moles de CO}] \text{ moins } [0,5\times\text{moles de } H_2].$$

fig-1

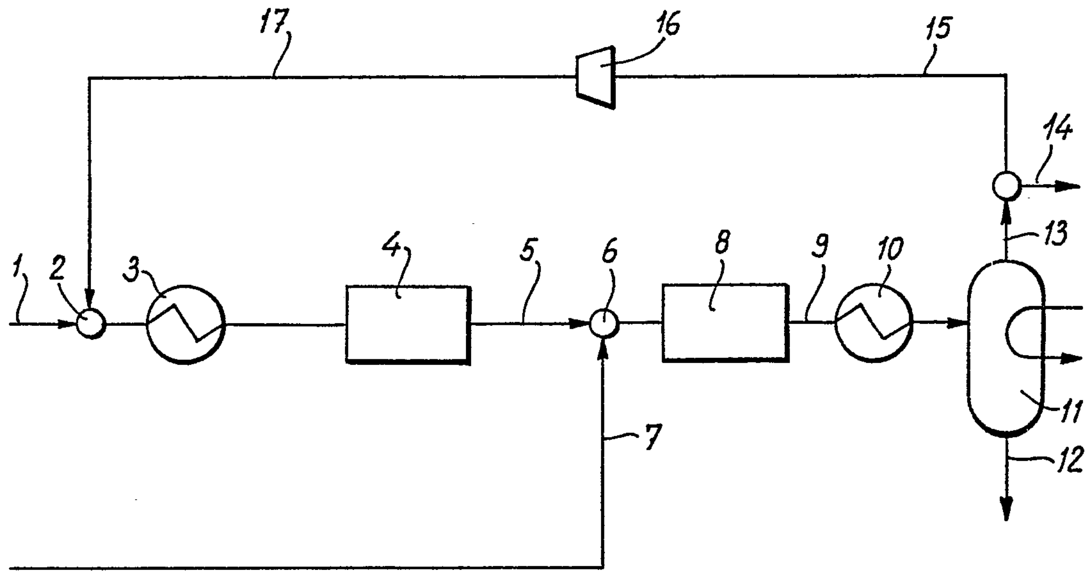

Fig-2

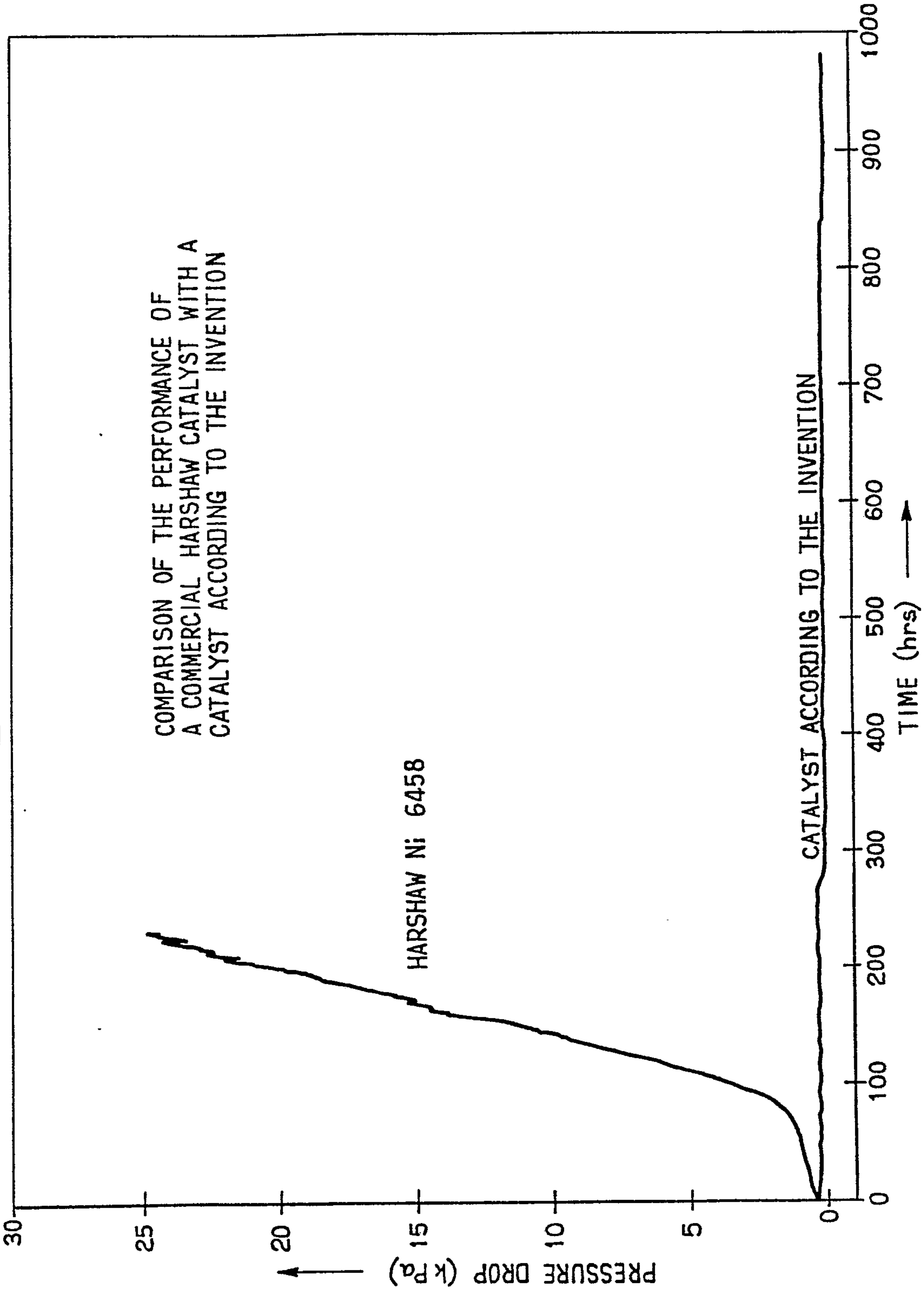
COMPARISON OF THE PERFORMANCE OF
A COMMERCIAL HARSHAW CATALYST WITH A
CATALYST ACCORDING TO THE INVENTION
HARSHAW Ni 6458
CATALYST ACCORDING TO THE INVENTION
PRESSURE DROP (k Pa)
0
5
10
15
20
25
30
TIME (hrs)
0
100
200
300
400
500
600
700
800
900
1000

Fig-3